# EUROPEAN PATENT APPLICATION

(11) **EP 2 537 889 A1**
(43) Date of publication of application: **26.12.2012**
(21) Application number: 11744518.9
(22) Date of filing: 03.02.2011
(51) Int. Cl.: C08K 5/357, C08L 23/08, C08L 101/00, C07D 251/34

(54) **CROSSLINKING AGENT FOR CROSSLINKABLE ELASTOMER, AND APPLICATION THEREOF**

(30) Priority: 17.02.2010 JP 2010032466
(71) Applicant: Nippon Kasei Chemical Company Limited, Iwaki-shi Fukushima 971-8101 (JP)
(72) Inventor: YAMAURA, Mabuko, Iwaki-shi Fukushima 971-8101 (JP); ORIKASA, Yukio, Iwaki-shi Fukushima 971-8101 (JP); SENZAKI, Kazuya, Iwaki-shi Fukushima 971-8101 (JP); KAGAWA, Takashi, Iwaki-shi Fukushima 971-8101 (JP)
(74) Representative: HOFFMANN EITLE
(86) International application number: PCT/JP2011/052248
(87) International publication number: WO 2011/102230

(57) **Abstract**

The object of the present invention is to provide a cross-linking agent for a cross-linkable elastomer which is rapid in the cross-linking rate in comparison with triallyl isocyanurate (TAIC).

The present invention relates to a cross-linking agent for a cross-linkable elastomer comprising a bis-isocyanurate derivative represented by the general formula (I). 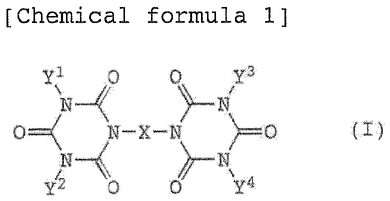 (In the formula, X represents the C1 to C12 alkenylene group or C1 to C12 alkylene group, which may contain a carbonyl group, an ether bond or an ester bond in the main chain, and further a substituent in the side chain; at least two of Y¹, Y², Y³ and Y⁴ are each independently an allyl group which may be substituted, and the rest is a hydrogen atom or a hydrocarbon group which may be substituted.)

## Description

### TECHNICAL FIELD

The present invention relates to a cross-linking agent for a cross-linkable elastomer and application thereof. More specifically, the present invention relates to a cross-linking agent for a cross-linkable elastomer comprising a bis-isocyanurate derivative having a specific structure, and application thereof. In the present invention, the term of cross-linkable elastomer means an elastomer having an active site which can be cross-linked by radical generation.

### BACKGROUND ART

Isocyanurate derivatives, especially triallyl isocyanurate (hereinafter referred as TAIC) is known as a cross-linking agent useful in obtaining a molded product by curing the cross-linkable elastomer.

However, although the cross-linkable elastomer molded product using TAIC is excellent in the chemical resistance and compression permanent strain, if TAIC is used for a substituted polyolefin, especially an ethylene-vinyl acetate copolymer, there is a disadvantage that the time of cross-linking process become longer because of slow cross-linking rate.

On the other hand, as a useful raw material for a curable-type resin, di(isocyanuric)-type compounds (bis isocyanurate derivatives) have been proposed (Patent Document 1).

However, in the above proposal, there is no mention for the cross-linkable elastomer.

### Prior Documents

### Patent Documents

Patent Document 1: Japanese Patent Application Laid-Open (KOKAI) No. 54-103881

### SUMMARY OF THE INVENTION

### PROBLEMS TO BE SOLVED BY THE INVENTION

The present invention has been made in view of the above circumstances. The object of the present invention is to provide a cross-linking agent for a cross-linkable elastomer which is rapid in the cross-linking rate in comparison with TAIC.

### MEANS FOR SOLVING THE PROBLEMS

As a result of the present inventors' earnest study, it has been found that the present invention can be achieved easily by the above bis-isocyanurate derivative.

Thus, in a first aspect of the present invention, there is provided a cross-linking agent for a cross-linkable elastomer comprising a bis-isocyanurate derivative represented by the general formula (I).

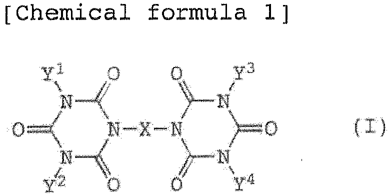

(In the formula, X represents the C1 to C12 alkenylene group or C1 to C12 alkylene group, which may contain a carbonyl group, an ether bond or an ester bond in the main chain, and further a substituent in the side chain; at least two of Y¹, Y², Y³ and Y⁴ are each independently an allyl group which may be substituted, and the rest is a hydrogen atom or a hydrocarbon group which may be substituted.)

In a second aspect of the present invention, there is provided a cross-linkable elastomer composition comprising a cross-linkable elastomer and the bis-isocyanurate derivative represented by the general formula (I), the blending amount of triazine derivative or prepolymer thereof being 0.05 to 15 parts by weight based on 100 parts by weight of the cross-linkable elastomer.

In a third aspect of the present invention, there is provided a process for producing an elastomer molded product by curing a cross-linkable elastomer, comprising using the bis-isocyanurate derivative represented by the general formula (I) as a cross-linking agent.

In a fourth aspect of the present invention, there is provided an elastomer molded product cured by the action of cross-linking agent, produced by using the bis-isocyanurate derivative represented by the general formula (I) as a cross-linking agent.

### EFFECT OF THE INVENTION

According to the present invention, there is provided a cross-linking agent for a cross-linkable elastomer which is rapid in the cross-linking rate in comparison with TAIC.

### BEST MODE FOR CARRYING OUT THE INVENTION

The present invention will be described in detail below.

### <Cross-linking agents for cross-linkable elastomer>

The cross-linking agents for cross-linkable elastomer according to the present invention comprises a bis-isocyanurate derivative represented by the above general formula (I).

As the hydrocarbon group which may be substituted in the above general formula (I), there are exemplified an aliphatic hydrocarbon group, an aromatic hydrocarbon group and the alicyclic hydrocarbon group which have 1 to 10 carbon atoms. Aliphatic hydrocarbon group also may have a branched structure which may have a substituent. As specific examples of the hydrocarbon group, there are exemplified an alkyl group, alkenyl group, an alkoxy group, a thioalkyl group, an alkoxycarbonyl group, a cyclohexyl group, phenyl group, benzyl group or the like. In addition, Y¹, Y², Y³ and Y⁴ in the formula (I) may be the same or different each other.

As concrete compounds of bis-isocyanurate derivative represented by general formula (I), there are exemplified 1,2-methylene-bis(3,5-diallyl isocyanurate), 1,2-ethylene-bis(3,5-diallyl isocyanurate), 1,3-propylene-bis(3,5-diallyl isocyanurate), 1,4-butylene-bis(3,5-diallyl isocyanurate), 1,5-pentyl-bis(3,5-diallyl isocyanurate), 1,6-hexane-bis(3,5-diallyl isocyanurate), 1,7-heptane-bis(3,5-diallyl isocyanurate), 1,8-octane-bis(3,5-diallyl isocyanurate), 1,10-decane-bis(3,5-diallyl isocyanurate), 1,12-dodecane-bis(3,5-diallyl isocyanurate), 1,2-propylene-bis(3,5-diallyl isocyanurate), 1,4-2-butene-bis(3,5-diallyl isocyanurate), 1,5-hexafluoro-pentyl-bis(3,5-diallyl isocyanurate) or the like.

The above bis-isocyanurate derivative is used as the cross-linking agent for the cross-linkable elastomer according to the present invention and the types and usage of cross-linkable elastomer will be described in the later description for the other invention.

### <Cross-linkable elastomer composition>

The cross-linkable elastomer composition according to the present invention is prepared by blending at least the bis-isocyanurate derivative represented by the above general formula (I) into the cross-linkable elastomer. The blending amount of bis-isocyanurate derivative is 0.05 to 15 parts by weight based on 100 parts by weight of cross-linkable elastomer.

The kind of cross-linkable elastomer is not particularly limited, for example, natural rubber, isoprene rubber, butadiene rubber, ethylene propylene rubber, styrene rubber, nitrile rubber, hydrogenated nitrile rubber, chloroprene rubber, chlorosulfonated polyethylene, acrylic rubber, ethylene acrylic rubber, silicone rubber, fluorine rubber, hydrin rubber or the like may be mentioned. In addition, there are exemplified substituted olefins which is a copolymer of olefin such as ethylene and propylene with vinyl alcohol, acrylic acid, methacrylic acid, ethyl acrylate, glycidyl methacrylate, vinyl acetate or the like. Further, a blended rubber comprising two or more components mentioned above may be also used. Of these, the substituted polyolefin is preferred. The type of substituted polyolefin is not particularly limited, and preferred thereof is ethylene-vinyl acetate copolymer. The vinyl acetate content in the ethylene-vinyl acetate copolymer is usually 10 to 40% by weight, preferably 20 to 35% by weight.

The blending ratio of bis-isocyanurate to the cross-linkable elastomer is preferably 0.05 to 15% by weight.

In the present invention, the other cross-linking agent may be used in combination with the above-mentioned cross-linking agent. The other cross-linking agents include, but are not particularly limited, an isocyanurate derivative represented by the following general formula (II) is preferred.

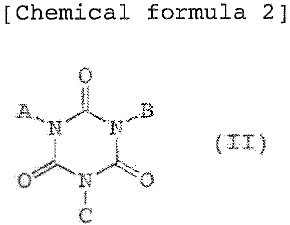

(In the formula (II), at least two of A, B, and C each independently represent an allyl group which may be substituted, and the rest represents a hydrogen atom or a hydrocarbon group which may be substituted.)

The above hydrocarbon group is synonymous with those described by the above general formula (I). In addition, A B and C in the formula (II) may be the same or different from each other.

The isocyanurate derivative represented by the general formula (II) has been already known. As specific examples thereof, there are exemplified triallyl isocyanurate (TAIC), diallylmethallyl isocyanurate, diallylbenzyl isocyanurate, diallyl-4-trifluoromethylbenzyl isocyanurate, tri-methallyl isocyanurate, diallylmethyl isocyanate, ethoxycarbonyl methyldiallyl isocyanate or the like.

When using the above bis-isocyanurate derivative and the above isocyanurate derivative in combination, the total amount thereof is usually 0.05 to 15 parts by weight, preferably from 0.5 to 5 parts by weight based on 100 parts by weight of the cross-linkable elastomer. The percentage of isocyanurate derivative to the total of all cross-linking agent is usually 5 to 95% by weight.

Further, in addition to the above isocyanurate derivative, polyfunctional (meth)acrylate may be used in combination. The polyfunctional (meth)acrylate has two or more, preferably three or more (meth)acryloyl groups in one molecule. More specifically, there are exemplified trimethylolpropane tri(meth)acrylate, pentaerythritol tri(meth)acrylate, tris((meth)acryloxyethyl) isocyanurate, dimethylolpropane tetra(meth)acrylate, tetraethylene glycol di(meth)acrylate, nonaethylene glycol di(meth)acrylate or the like. The amount of these polyfunctional (meth)acrylate added is 0.05 to 15 parts by weight based on 100 parts by weight of cross-linkable elastomer.

To the cross-linkable elastomer composition, an organic peroxide may be blended. The organic peroxide is usually an essential component for the heat cross-linking, and is not particularly limited as long as it is a known organic peroxide to generate peroxy radicals under the vulcanization conditions. There are exemplified di-t-butylperoxide, dicumylperoxide, 2,5-dimethyl-2,5-di(benzoylperoxy) hexane, 2,5-dimethyl-2,5-di(t-butylperoxy) hexane, t-butylperoxy-2-ethylhexyl-monocarbonate, 1,1-bis(t-butylperoxy)-3,5,5-trimethyl cyclohexane, 2,5-dimethyl-2,5-dihydroxyperoxide, t-butylcumylperoxide, α, α'-bis(t-butylperoxy)-p-diisopropyl benzene, 2,5-dimethyl-2,5-di(t-butylperoxy) hexyne, benzoylperoxide, t-butylperoxy benzene or the like.

The blending amount of the organic peroxide, may vary depending on the type of used cross-linkable elastomer, and is usually 0.1 to 10% by weight, preferably 0.5 to 5% by weight based on 100 parts by weight of the cross-linkable elastomer. In case of radiation cross-linking, the organic peroxide is not necessarily required.

In the present invention, known additives such as a polymerization inhibitor, filler, pigment, stabilizer, lubricant, releasing agent, plasticizer, anti-aging agent, silane coupling agent, ultraviolet absorber, flame retardant and acid acceptor can be used.

As the anti-aging agent, there are exemplified di-t-butyl-P-cresol, pentaerythrityl-tetraxy[3-(3,5-di-t-butyl-4-hydroxyphenyl)propionate], 2,2'-methylenebis(2-methyl-6-t-butylphenyl), bis(2,2,6,6-tetramethyl-4-piperadyl)sebacate, N,N'-hexane-1,6-diylbis[3-(3,5-di-tert-butyl-4-hydroxyphenyl)propionamido], bis(2,2,6,6-tetramethyl-4-piperadyl)sebacate, hydroquinone monomethylether, methylhydroquinone or the like.

As the silane coupling agent, there are exemplified γ-chloropropyl trimethoxysilane, vinyl triethoxysilane, vinyl-tris-(β-methoxyethoxy) silane, γ-methacryloxypropyl trimethoxysilane, β-(3,4-ethoxy-cyclohexyl)ethyl trimethoxysilane, γ-glycidoxypropyl trimethoxysilane, γ-mercaptopropyltrimethoxysilane, γ-aminopropyl trimethoxysilane, N-β-(aminoethyl)-γ-aminopropyl trimethoxysilane or the like.

As the ultraviolet absorber, there are exemplified 2-hydroxy-4-n-octyloxy benzophenone, 2,2-hydroxy-4,4-dimethoxy benzophenone, 2-(2'-hydroxy-5-methylphenyl) benzotriazole, p-t-butylphenyl salicylate or the like.

The blending amount of the above additive is usually not more than 10 parts by weight, preferably 5 parts by weight based on 100 parts by weight of the cross-linkable polymer.

Each of the above ingredients are mixed by a usual kneader such as a Banbury mixer, a kneader, an open roll or the like to form a cross-linkable elastomer composition.

### <Process for producing an elastomer molded product>

The process according to the present invention is a process for producing an elastomer molded product by curing the cross-linkable elastomer. Then, as the cross-linking agent, the bis-isocyanurate derivative represented by the above general formula (I) is used. As the cross-linking, either heating cross-linking and radiation cross-linking may be used, heating cross-linking is preferred.

The heating cross-linking is conducted by such a manner that, after filling a prescribed amount of cross-linkable elastomer composition is filled in a mold having a desired shape, and subjected to primary cross-linking by a heating process, if necessary, secondary cross-linking is applied in the oven. The shape of mold in the molding machine can be optionally selected from, for example, a sheet-shape, rod-shape, ring-shape and various complex block-shapes depending on the application of obtained elastomer molded product.

The primary cross-linking is conducted by heating thereof at usually 120 to 200°C for 2 to 30 minutes by use of, for example, an injection molding machine, pressurizing molding machine or the like.

The secondary cross-linking is conducted at 120 to 200°C for 1 to 10 hours.

In addition, as the radiation used in the radiation cross-linking, there ca be used an electron beam acceleration, X-ray, α-ray, β-ray, γ-ray or the like. The irradiation dose may vary depending on the used cross-linkable elastomer type is usually 0.1 to 500 kGy.

### <Elastomer molded product>

The elastomer molded product according to the present invention is an elastomer molded product cured by the action of cross-linking agent. As the cross-linking agent, the bis-isocyanurate derivative represented by the above general formula (I) is used. The process for producing thereof is as described above.

### EXAMPLES

The present invention is described in more detail below by the following Examples. However, these Examples are only illustrative and not intended to limit the present invention thereto unless they depart from the scope of the present invention. Evaluation methods used in the following Examples are shown as follows.

### Synthesis Example 1 [Synthesis of 1,2-ethylene-bis-(3,5-diallyl isocyanurate)]

After 12.0g (0.05 mol) of diallyl isocyanurate and 8.4g (0.06 mol) of potassium carbonate were added into 60g of N,N-dimethylformamide (DMF) and dissolved, 3.0g (0.03 mol) of 1,2-dichloroethane was added thereinto and the reaction was conducted at the temperature of 120°C for 3 hours. Thereafter, the reaction mixture was cooled and filtered to remove the inorganic product. The obtained filtrate was distilled under reduced pressure to recover the solvent. The obtained residue was diluted with ethyl acetate, washed with 5% by weight of aqueous hydrochloric acid solution, washed with water and thereafter, dried by anhydrous magnesium sulfate and filtered. The obtained filtrate is distilled under reduced pressure to recover the ethyl acetate contained therein. Further, into the residue, 50g of isopropyl alcohol was added with stirring to precipitate the objective product. The obtained precipitate was filtered and dried to obtain 10.6g of 1,2-ethylene-bis-(3,5-diallyl isocyanurate as a powder product (LC purity: 95%, yield: 88%)

The above "LC purity" was determined as an area percentage by conducting liquid chromatography measurement where an "INERTSIL ODS-3" column (25cm) was set to "LC-10ADVP" manufactured by Shimadzu Corporation and a mixed solvent of acetonitrile and water was used.

### Examples 1 to 3 and Comparative Example 1:

By using an open roll, respective components shown in Table 1 were kneaded with ethylene-vinyl acetate copolymer (EVA) in amounts of respective components shown in Table 1. The obtained composition was subject to heat-press at 150°C to obtain a sheet having 1 mm thickness.

When kneading above, by using a Curelastometer, the torque of composition (150°C) was read the value over 15 minutes to measure the cross-linking rate. The intermediate value of maximum torque value and minimum torque value was "torque when cross-linking rate is 50%" and the time requiring therefor is "time when the cross-linking percentage is reached to 50%". Then, the value of 30% of the difference between the maximum torque value and minimum torque value was "torque when cross-linking rate is 30%" and the time requiring therefor is "time when the cross-linking percentage is reached to 30%". The evaluation results are shown in Table 2.

**[Table 1]**

| Compounding (parts by weight) | Example 1 | Example 2 | Example 3 | Comp. Example 1 |
|---|---|---|---|---|
| EVA⁽¹⁾ | 100 | 100 | 100 | 100 |
| Organic peroxide⁽²⁾ | 1.3 | 1.3 | 1.3 | 1.3 |
| Cross-linking agent- 1⁽³⁾ | 2.0 | 0.2 | 0.5 | - |
| Cross-linking agent-2⁽¹⁾ | - | 1.8 | 1.8 | 2.0 |
| Silane coupling agent⁽⁵⁾ | 0.5 | 0.5 | 0.5 | 0.5 |
| Ultra violet absorber⁽⁶⁾ | 0.2 | 0.2 | 0.2 | 0.2 |

| | | | | |
|---|---|---|---|---|
| (1) Vinyl acetate content: 26% by weight (2) 2,5-dimethyl-2,5-(t-butylperoxy)hexane (manufactured by NOF Corporation (3) 1,2-ethylene-bis(3,5-diallyl isocyanurate) (4) Triallyl isocyanurate (manufactured by Nippon Kasei Chemical Co., Ltd.) (5) γ-methacryloxy propyltrimethoxy silane (6) 2-hydroxy-4-n-benzophenone octoxyphenyl | | | | |

**[Table 2]**

| Evaluation results | Example 1 | Example 2 | Example 3 | Comp. Example 1 |
|---|---|---|---|---|
| Torque when cross-linking rate is 50% (dNm) | 3.0 | 3.0 | 3.0 | 3.0 |
| Time when the cross-linking percentage is reached to 50 (min) | 7.1 | 7.4 | 7.1 | 7.7 |
| Torque when cross-linking rate is 30% (dNm) | 1.9 | 1.9 | 1.9 | 1.9 |
| Time when the cross-linking percentage is reached to 30% (min) | 5.9 | 6.5 | 6.2 | 6.7 |

### INDUSTRIAL APPLICABILITY

The molded product of cross-linkable elastomer composition according to the present invention using the ethylene-vinyl acetate copolymer as the polyolefin substituted is useful for applications such as: packaging materials for various materials such as foods, pharmaceuticals, industrial chemicals and agricultural chemicals, various adhesive films sealing films for solar cells, as well as useful in fields of hemodialysis, plasma component separation, desalting of protein solutions, fractionation, condensation, condensation of fruit juice and wastewater treatment.

## Claims

1. A cross-linking agent for a cross-linkable elastomer comprising a bis-isocyanurate derivative represented by the general formula (I). 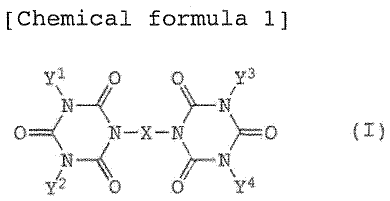 (In the formula, X represents the C1 to C12 alkenylene group or C1 to C12 alkylene group, which may contain a carbonyl group, an ether bond or an ester bond in the main chain, and further a substituent in the side chain; at least two of Y¹, Y², Y³ and Y⁴ are each independently an allyl group which may be substituted, and the rest is a hydrogen atom or a hydrocarbon group which may be substituted.)

2. A cross-linking agent according to claim 1, wherein the cross-linkable elastomer is a substituted polyolefin.

3. A cross-linking agent according to claim 2, wherein the polyolefin is substituted ethylene-vinyl acetate copolymer.

4. A cross-linkable elastomer composition comprising a cross-linkable elastomer and the bis-isocyanurate derivative represented by the general formula (I) as defined in claim 1, the blending amount of triazine derivative or prepolymer thereof being 0.05 to 15 parts by weight based on 100 parts by weight of the cross-linkable elastomer.

5. A cross-linkable elastomer composition according to claim 4, wherein the cross-linkable elastomer is a substituted polyolefin.

6. A cross-linkable elastomer composition according to claim 5, wherein the substituted polyolefin is an ethylene-vinyl acetate copolymer.

7. A process for producing an elastomer molded product by curing a cross-linkable elastomer, comprising using the bis-isocyanurate derivative represented by the general formula (I) as defined in claim 1 as a cross-linking agent.

8. A process according to claim 7, wherein the cross-linkable elastomer is a substituted polyolefin.

9. A process according to claim 8, wherein the substitution polyolefin is an ethylene-vinyl acetate copolymer.

10. An elastomer molded product cured by the action of cross-linking agent, produced by using the bis-isocyanurate derivative represented by the general formula (I) as defined in claim 1 as a cross-linking agent.

11. An elastomer molded product according to claim 10, wherein the elastomer is a substituted polyolefin.

12. An elastomer molded product according to claim 11, wherein the substituted polyolefin is an ethylene-vinyl acetate copolymer.
